# EUROPEAN PATENT SPECIFICATION

(11) **EP 4 188 118 B1**
(45) Date of publication and mention of the grant of the patent: **25.12.2024**
(21) Application number: 21758440.8
(22) Date of filing: 30.07.2021
(51) Int. Cl.: A61K 31/164, A23L 5/00, A61K 31/19, A61P 1/00, A61P 1/04, A61P 29/00

(54) **ASSOCIATION OF BUTYRATE AND PALMITOYLETHANOLAMIDE FOR THE TREATMENT OF INFLAMMATORY BASED INTESTINAL DISORDERS**
ASSOZIATION VON BUTYRAT UND PALMITOYLETHANOLAMID ZUR BEHANDLUNG VON ENTZÜNDLICHEN DARMERKRANKUNGEN
ASSOCIATION DE BUTYRATE ET DE PALMITOYLÉTHANOLAMIDE POUR TRAITER DES TROUBLES INTESTINAUX INFLAMMATOIRES

(30) Priority: 31.07.2020 IT 202000018703
(43) Date of publication of application: 07.06.2023
(73) Proprietor: Unifarco S.p.A., 32035 Santa Giustina (BL) (IT)
(72) Inventor: BARATTO, Giovanni, 32035 Santa Giustina BL (IT)
(74) Representative: Perani & Partners S.p.A.
(86) International application number: PCT/IB2021/056960
(87) International publication number: WO 2022/024057

(56) References cited:
- RUSSO ROBERTO ET AL: "Gut-brain Axis: Role of Lipids in the Regulation of Inflammation, Pain and CNS Diseases", vol. 25, no. 32, 16 October 2018 (2018-10-16), NL, pages 3930 - 3952, XP055794155, ISSN: 0929-8673, Retrieved from the Internet <URL:https://eurekaselect.com/article/download/150145> DOI: 10.2174/0929867324666170216113756
- FRANCESCA BORRELLI ET AL: "Palmitoylethanolamide, a naturally occurring lipid, is an orally effective intestinal anti-inflammatory agent : Palmitoylethanolamide and colitis", BRITISH JOURNAL OF PHARMACOLOGY, vol. 172, no. 1, 1 December 2014 (2014-12-01), UK, pages 142 - 158, XP055523214, ISSN: 0007-1188, DOI: 10.1111/bph.12907
- KELLY CALEB J ET AL: "Crosstalk between Microbiota-Derived Short-Chain Fatty Acids and Intestinal Epithelial HIF Augments Tissue Barrier Function", CELL HOST & MICROBE, ELSEVIER, NL, vol. 17, no. 5, 9 April 2015 (2015-04-09), pages 662 - 671, XP029169483, ISSN: 1931-3128, DOI: 10.1016/J.CHOM.2015.03.005

## Description

### FIELD OF THE INVENTION

The field of the present invention relates to a combination for use in treating disorders and pathologies of the intestinal tract associated with or caused by inflammatory processes.

### BACKGROUND ART

The wall of the human gastrointestinal tract constitutes one of the major areas of interaction between our body and the external environment and plays a fundamental role in the regulation of the immune system, and therefore in the maintenance of the state of health of a body. The intestinal barrier is semi-permeable and is responsible for the absorption of nutrients, immune detection, and defence against potentially harmful antigens as well as pathogenic microorganisms [1]. In particular, the intestinal epithelium contains: absorbent cells, secretory cells, and intestinal stem cells. The absorbent cells are distinguished in: enterocytes, in the small intestine tract, and colonocytes, in the colon tract. The secretory cells include goblet cells (or GC), Paneth cells, and enteroendocrine cells. The enterocytes and colonocytes are the most-present cell types in the intestinal epithelium, with a relative abundance exceeding 80%. In addition to the physical barrier function between the immune system and the commensal microbiota, the enterocytes and epithelial colonocytes are of key importance for the absorption of water as well as nutrients (mainly the role of the enterocytes) and electrolytes (essentially the function of the colonocytes) [2] [1].

The first line of physical defence which prevents bacteria in the intestinal lumen from coming into direct contact with intestinal epithelial cells is the mucous layer of the intestinal tract. The intestinal mucous layer consists mainly of mucins, i.e., highly glycosylated proteins, which form a gelatinous structure (mucus). The mucus covers and protects the intestinal epithelium. For example, the most abundant mucin in the intestine is MUC-2 (Mucin 2, Oligomeric Mucus/Gel-Forming), which is secreted by the goblet cells [3] [1] [4].

### Intestinal oxygen gradient

Under physiological conditions, the intestinal wall is characterized by an oxygen gradient with values around 8% in the lamina propria, which are reduced to 3% at the intestinal villi, and less than 2% in the lumen. Therefore, considering the oxygen gradient and the daily fluctuations that make the microenvironment extremely complex, it can be said that the intestinal mucosa is in a state of physiological hypoxia [5] [6].

Adaptation to oxygen fluctuations depends on the activation of the HIF-1 (Hypoxia Inducible Factor) cascade. Under hypoxic conditions, the HIF-1 factor is activated at the nuclear level, which triggers a reprogramming of gene expression which controls the fate of the cell, generally activating alternative energy generation mechanisms and increasing oxygen uptake [7].

In particular, the activation of HIF-1 promotes:
- the expression of MUC-2 which, as mentioned, is the main mucin constituting the mucous layer of the intestinal wall [8] [9];
- the expression of ITF (Intestinal Trefoil Factor), a peptide involved in epithelial reconstruction and protection processes [10].

### Disorders associated with alterations of the intestinal barrier

It is commonly assumed that a dysfunction of the intestinal barrier and an uncontrolled flow of antigens through the intestinal epithelium can constitute a threat to the immune system of susceptible individuals, and affect the balance between host and microbiota, triggering inflammatory mechanisms in the intestine and/or other districts of the body [1]. Environmental factors such as stress, diet, lifestyle, smoking habits, and changes in the intestinal bacterial component may be further associated with an altered immune response [11].

The alteration of intestinal barrier integrity favours the development of sometimes exaggerated immune responses which are associated with the development of inflammatory diseases of the gastrointestinal tract. Under normal physiological conditions, increased permeability is usually not sufficient to cause intestinal disease, since the epithelial barrier can regenerate and regain its functions once the injurious stimulus disappears. However, under certain pathological conditions, this ability to self-regulate may be lost, and may contribute to increased permeability, thereby facilitating chronicization of intestinal inflammation. The presence of certain environmental factors (infections, stress) increases intestinal permeability and the passage of substances which under normal conditions would never penetrate the epithelial barrier [12] [13].

IBD (Inflammatory Bowel Diseases, or ulcerative colitis and Crohn's disease) and IBS (Irritable Bowel Syndrome) are gastrointestinal disorders which affect 0.5% and 21% of the world population, respectively. The two pathological conditions have similar symptoms with the difference that IBD is characterized by an inflammatory state generally found with endoscopic examination; while IBS is considered a functional disease without objectively measurable structural or physiological abnormalities, thus making its diagnosis more difficult [14].

Numerous studies in the literature on dysbiosis and IBD have shown the role of the intestinal microbiota as an important cause of intestinal inflammation. In addition to genetic and environmental factors, altered barrier function also leads to an abnormal immune response and increased susceptibility to intestinal inflammation. In fact, although the aetiology of IBD and IBS remains unknown, all patients share an increased intestinal permeability with respect to healthy subjects. Increased intestinal permeability is the result of structural changes in the proteins that make up tight junctions (or TJ), and would result from an exaggerated inflammatory response. An increase in IFN-γ and TNF-α levels was indeed detected in patients with IBD or IBS [15] [16] [17] [18] [19] [11].

Even in celiac disease, an autoimmune condition of unknown etiology which derives from gluten intolerance, structural changes of the TJ are found, which favour the entry of gliadin (gluten constituent protein) into the intestinal mucosa [20] [21]. This results in a sustained immune response which contributes to increasing intestinal permeability. In general, the altered function of the intestinal barrier is also involved in the patho-physiology of food allergies. Increased intestinal permeability may promote the passage of food antigens and increase the risk of an allergic response in sensitive subjects. In fact, in patients with food allergies there is greater intestinal permeability, as measured by the lactulose and mannitol permeability test [22].

Butyrate is a short-chain saturated fatty acid (Fig. 1) produced by the fermentation of indigestible carbohydrates by some types of bacteria resident in the colon; as a product of intestinal microbiota it is considered a postbiotic. Butyrate is the primary energy substrate for colonocytes and is critical for the maintenance of intestinal epithelial homoeostasis [23] [24].

Butyrate is transported from the intestinal lumen to the cytosol of the colonocyte largely actively, to then diffuse into the mitochondria and be oxidized with CO₂ release, ATP regeneration and H₂O production from O₂ (aerobic respiration) [25]. The consumption of O₂ within the colonocyte results in a state of physiological hypoxia which acts as an activation signal for HIF-1 (Hypoxia Inducible Factor) [10] [26] [27] [28]. As described above, the HIF-1 factor promotes the expression of MUC-2 and ITF, favouring the protection of the intestinal wall [8] [10] [9]. In addition, butyrate:
- promotes the assembly of tight junctions (TJ) contributing to the reduction of intestinal permeability [29];
- inhibits the activation of the transcription factor NF-kB (Nuclear Factor kappa-light-chain-enhancer of activated B cells) and the enzyme HDAC (Histone deacetylases) by counteracting inflammation and cell proliferation [30] [31] [32] [33] [34].

The daily dose of sodium butyrate used in clinical trials varies by application, from 300 mg to 4 g. The main competitor recommends up to 1.5 g of sodium butyrate for intestinal well-being [35] [36] [37] [38] [39].

Palmitoylethanolamide (or PEA) is a lipid mediator (Fig. 2) belonging to the family of *N*-acylethanolamines to which neuroprotective, anti-inflammatory and analgesic properties are attributed. PEA is found in many foods in nature, such as legumes, egg yolks and peanuts, but is also present in the tissues of animals and humans [40] [41] [42]. PEA represents a PPAR-α agonist with affinity comparable to Wy-14643 (synthetic receptor agonist) which produces a strong anti-inflammatory effect. The binding of PEA to PPAR-α induces heterodimerization between PPAR-α and RXR (Retinoid X Receptor) and the formation of the activated receptor complex. Thereby, the receptor is formed in the active form which translocates into the nucleus where it binds the peroxisome proliferation response elements. Accordingly, it thereby reduces the transcription of pro-inflammatory genes. In addition, as an entourage effect, PEA could activate target endocannabinoid receptors, i.e., CB1 and CB2 receptors and TRPV1 (transient receptor potential vanilloid receptor type 1) channels, exerting a pain-relieving effect. Theoretically, the analgesic action of endocannabinoids, arachidonoylglycerol and anandamide, depends on the levels of PEA production. In pain conditions, the endogenous expression of PEA is usually reduced and that of arachidonoylglycerol and anandamide increases. However, the increase in the latter is not such as to exert an analgesic action due to the low PEA production levels. Therefore, the supplementation of PEA could represent an effective alternative to enhance endogenous anti-nociceptive mechanisms exerted by endocannabinoids[41] [43] [40].

*In vitro*, *in vivo*, and *ex vivo* studies demonstrate the utility of PEA in combating IBD-associated chronic intestinal inflammation. In fact, the PEA seems to be able to:
- improve the macroscopic signs of ulcerative colitis (edema and mucosal erosion);
- reduce expression and release of pro-inflammatory cytokines (such as NO, PGE2, TNF-α) and expression of iNOS and COX2;
- limit the activation and infiltration of neutrophils and macrophages;
- reduce angiogenesis;
- reduce the weight and length of the intestinal loops;
- re-equilibrate the correct intestinal permeability;
- promote the regeneration of the affected area;
- reduce splenomegaly;
- improve the severity of abdominal pain and the parameters of the DAI (disease activity index) scale [44] [45] [46].

A recent clinical study of 54 patients with IBS showed that the administration of 200 mg PEA and 20 mg polydatin markedly improved abdominal pain severity but did not impact its frequency [47].

The daily dose of PEA used in clinical trials to control pain ranges from 300 mg to 1.2 g. The daily dose most commonly found in the works is 600 mg. There is no statistically significant association between PEA dose and efficacy in counteracting pain. Similarly, the duration of treatment, ranging from 10 to 180 days, does not appear to significantly impact the desired outcome. In addition, on the whole, PEA treatment is well tolerated [48].

Generally the condition of intestinal inflammatory state can be treated therapeutically, for example, with the use of NSAID anti-inflammatory drugs or of those of a steroidal nature. However, such drugs have no effect in reducing the permeability of the intestinal wall and are often the cause of undesirable adverse reactions, especially when the underlying administration is long-term.

Bioactive lipids such as palmitoylethanolamide (PEA) and short chain fatty acids (SCFAs), such as butyrate belong to a large group of bioactive lipids with anti-inflammatory effects in the gastrointestinal tract. PEA reduces inflammation markers in a murine model of inflammatory bowel disease (IBD), and butyrate, produced by gut microbiota, is effective in reducing inflammation and pain in irritable bowel syndrome and IBD animal models [52].

### SUMMARY OF THE INVENTION

The Applicant has advantageously developed a combination for use in treating and preventing disorders and pathologies of the intestinal tract associated with or caused by inflammatory processes comprising the following active ingredients: a) sodium butyrate and b) palmitoylethanolamide (or PEA). Sodium butyrate is administered at daily dosages between 400 and 3000 mg daily and PEA is administered at daily dosages between 200 and 1200 mg daily wherein the weight ratio between sodium butyrate and PEA administered daily is comprised between 2:1 and 6:1.

The combination developed by the Applicant is based on the restoration of barrier integrity by acting in a targeted and parallel manner on two levels:
- the reduction of intestinal permeability (leaky gut), through the use of sodium butyrate with consequent reduction of the access of harmful non-self substances and microorganisms;
- limiting the inflammatory state (immune system reactivity) and nociception, through the use of palmitoylethanolamide (or PEA) with consequent modulation of the inflammatory factors which contribute to the alteration of the barrier.

The action on two distinct levels in parallel allows to stop the vicious cycle of inflammation, allowing the intestinal tissues to find a homoeostasis condition.

Such a combination is advantageous because its administration in patients with disorders or pathologies associated with or caused by inflammatory processes allows to reduce the healing times compared to the administration of the individual active ingredients sodium butyrate a) and PEA b).

Finally, said combination is well tolerated and does not present any adverse effect.

### DETAILED DESCRIPTION OF THE INVENTION

For the purposes of the present invention, the verbs "comprise" or "contain" are intended to define a set of elements, expressly indicating some, without excluding the presence of others not expressly indicated; while the term "substantial" or "constituted" is intended to define a set of elements, expressly indicating them all, and thus excluding the presence of components not expressly listed.

The combination developed by the Applicant is for use in treating disorders or pathologies of the intestinal tract associated with or caused by inflammatory processes. Said disorders or pathologies of the intestinal tract associated with or caused by inflammatory processes are preferably selected from: irritable bowel syndrome (IBS), inflammatory bowel disease (IBD), leaky bowel syndrome, celiac disease and food allergies.

For the claimed combination, the weight ratio of sodium butyrate to PEA is preferably comprised between 2:1 and 5:1, more preferably is equal to 3:1.

Said combination is preferably administered in the form of two oral formulations, of which the first oral formulation contains sodium butyrate as active ingredient while the second oral formulation contains PEA as active ingredient, or said combination is preferably administered in the form of a single oral formulation containing both the active ingredients sodium butyrate a) and PEA b).

When said combination is preferably administered in the form of two oral formulations, of which the first oral formulation contains sodium butyrate as active ingredient and of which the second oral formulation contains PEA as active ingredient, preferably said second oral formulation containing PEA also contains vitamin B2.

When said combination is preferably administered in the form of two oral formulations, of which the first oral formulation contains sodium butyrate as active ingredient and of which the second oral formulation contains PEA as active ingredient, said first oral formulation more preferably contains sodium butyrate in an amount of 900 mg and more preferably said second oral formulation contains PEA in an amount of 300 mg, and both are preferably administered twice daily.

When said combination is preferably administered in the form of oral formulations, said oral formulations are preferably food supplements or nutraceutical formulations.

Food supplements are intended as a food product designed to supplement the common diet as they constitute a concentrated source of nutrients, such as vitamins and minerals, or of other substances having a nutritional or physiological effect, in particular, but not exclusively, amino acids, essential fatty acids, fibres and extracts of plant origin, both mono- and multi-compound, in predominant forms.

When said combination is preferably administered in the form of oral formulations, said oral formulations are preferably adjuvants.

Adjuvant is intended as a pharmacological substance which, added to a real drug, modifies its effect in order to enhance or maximize its effectiveness.

When said combination is preferably administered in the form of oral formulations, they contain the aforementioned active ingredients in combination with conventional excipients and which may possibly control and/or delay their release.

Said oral formulations are preferably in the form of tablets, capsules, sachets, stickpacks in liquid form or orodispersible or orosoluble powder form, bottles, ampoules.

### BIBLIOGRAPHY

[1] M. Vancamelbeke e S. Vermeire, «The intestinal barrier: a fundamental role in health and disease,» Expert Rev Gastroenterol Hepatol, vol. 11, n. 9, pp. 821-834, 2017.
[2] J. Spence, R. Lauf e N. Shroyer, «Vertebrate Intestinal Endoderm Development,» Dev Dyn, vol. 240, n. 3, pp. 501-520, 2011.
[3] M. Johansson, J. Larsson e G. Hansson, «The two mucus layers of colon are organized by the MUC2 mucin, whereas the outer layer is a legislator of host-microbial interactions,» PNAS, vol. 108, n. 1, pp. 4659-4665, 2011.
[4] M. Johansson, Henrik Sjövall, and Gunnar C, H. Sjövall e G. Hansson, «The gastrointestinal mucus system in health and disease,» Nat Rev Gastroenterol Hepatol, vol. 10, n. 6, pp. 352-361, 2013.
[5] L. Glover e S. Colgan, «Hypoxia And Metabolic Factors That Influence Inflammatory Bowel Disease Pathogenesis,» Gastroenterology, pp. 1748-1755, 2011.
[6] N. Zeitouni, S. Chotikatum, M. von Kockritz-Blickwede e al., «The impact of hypoxia on intestinal epithelial cell functions: consequences for invasion by bacterial pathogens,» Mol Cell Pediatr, 2016.
[7] M. Manresa e C. Taylor, «Hypoxia Inducible Factor (HIF) hydroxylases as regulators of intestinal epithelial barrier functions,» Cell Mol Gastroenterol Hepatol., pp. 303-315, 2017.
[8] A. Dilly, Y. Lee, H. Zeh e al, «Targeting Hypoxia-Mediated Mucin 2 Production as a Therapeutic Strategy for Mucinous Tumors,» Transl Res, vol. 169, pp. 19-30, 2016.
[9] N. Louis, K. Hamilton, G. Canny e al, «Selective induction of mucin-3 by hypoxia in intestinal epithelia,» J Cell Biochem, vol. 99, p. 1616-1627, 2006.
[10] G. Furuta, J. Turner, C. Taylor e al, «Hypoxia-inducible factor 1-dependent induction of intestinal trefoil factor protects barrier function during hypoxia,» J Exp Med, vol. 193, pp. 1027-1034, 2001.
[11] M. Santoru e al, «Cross sectional evaluation of the gut-microbiome metabolome axis in an Italian cohort of IBD patients,» Sci Reports, vol. 7, n. 9523, 2017.
[12] M. Rescigno, «The intestinal epithelial barrier in the control of homeostasis and immunity,» Trends Immunol, vol. 32, pp. 256-264, 2011.
[13] E. Salvo-Romero, C. Alonso-Cotoner e C. Pardo-Camacho, «The intestinal barrier function and its involvement in digestive disease,» Rev Esp Enferm Dig, vol. 107, n. 11, pp. 686-696, 2015.
[14] V. Arnau e al, «Gut microbiota composition and functional changes in inflammatory bowel disease and irritable bowel syndrome,» Sci Translational Med, vol. 10, n. 472, 2018.
[15] S. Prasad, R. Mingrino, K. Kaukinen e al, inflammatory processes have differential effects on claudins 2, 3 and 4 in colonic epithelial cells,» Lab Invest, vol. 85, pp. 1139-1162, 2005.
[16] S. Zeissig, N. Bürgel, D. Giinzel e al, «Changes in expression and distribution of claudin 2, 5 and 8 lead to discontinuous tight junctions and barrier dysfunction in active Crohn's disease,» Gut, vol. 56, pp. 61-72, 2007.
[17] S. Blair, S. Kane, D. Clayburgh e al, «Epithelial myosin light chain kinase expression and activity are upregulated in inflammatory bowel disease,» Lab Invest, vol. 86, pp. 191-201, 2006.
[18] L. Wang, N. Alammar, R. Singh e al, «Gut microbial dysbiosis in the irritable bowel syndrome: a systematic review and meta-analysis of case-control studies,» J Acad Nutr Diet, 2019.
[19] L. Ohman e M. Simren, «Pathogenesis of IBS: role of inflammation, immunity and neuroimmune interactions,» Nat Rev Gastroenterol Hepatol, vol. 7, n. 3, pp. 163-173, 2010.
[20] D. Pizzuti, M. Bortolami, E. Mazzon e al, «Transcriptional downregulation of tight junction protein ZO-1 in active coeliac disease is reversed after a gluten-free diet,» Dig Liver Dis, vol. 36, pp. 337-341, 2004.
[21] G. Sander, A. Cummins, T. Henshall e al, «Rapid disruption of intestinal barrier function by gliadin involves altered expression of apical junctional proteins,» FEES Lett, vol. 579, pp. 4851-4855, 2005.
[22] M. Ventura, L. Polimeno, A. Amoruso e al, «Intestinal permeability in patients with adverse reactions to food,» Dig Liver Dis, vol. 38, pp. 732-736, 2006.
[23] M. Cuff, J. Dyer, M. Jones e al, «The human colonic monocarboxylate transporter Isoform 1: its potential importance to colonic tissue homeostasis,» Gastroenterology, vol. 128, n. 3, pp. 676-686, 2005.
[24] A. Wachtershauser e J. Stein, «Rationale for the luminal provision of butyrate in intestinal diseases,» Eur JNutr, vol. 39, n. 4, pp. 164-171, 2000.
[25] D. Donohoe, N. Garge, X. Zhang e al, «The Microbiome and Butyrate Regulate Energy Metabolism and Autophagy in the Mammalian Colon,» Cell Metab, vol. 13, n. 5, pp. 517-526, 2011.
[26] A. Carreau, B. Rahbi e A. Matejuk, «Why Is the Partial Oxygen Pressure of Human Tissues a Crucial Parameter? Small Molecules and Hypoxia,» J Cell Mol Med, vol. 15, n. 6, pp. 1239-1253, 2011.
[27] F. Rivera-Chávez, C. Lopez e A. Bäumler, «Oxygen as a Driver of Gut Dysbiosis,» Free Radic Biol Med, vol. 105, pp. 93-101, 2017.
[28] C. Kelly, L. Zheng, E. Campbell e al, «Crosstalk between Microbiota-Derived Short-Chain Fatty Acids and Intestinal Epithelial HIF Augments Tissue Barrier Function,» Cell Host Microbe, vol. 17, n. 5, p. 662-671, 2015.
[29]L. Peng, Z. Li, R. Green e al, «Butyrate enhances the intestinal barrier by facilitating tight junction assembly via activation of AMP-activated protein kinase in Caco-2 cell monolayers,» J Nutr, vol. 139, pp. 1619-1625, 2009.
[30]J. Biddlestone, D. Bandarra e S. Rocha, «The role of hypoxia in inflammatory disease (Review),» Int J Mol Med, vol. 35, pp. 859-869, 2015.
[31]M. Thangaraju, G. Cresci, K. Liu e al, «GPR109A is a G-protein-coupled receptor for the bacterial fermentation product butyrate and functions as a tumor suppressor in colon,» Cancer Res, vol. 69, n. 7, pp. 2826-2832, 2009.
[32]R. Schilderink, C. Verseijden e W. de Jonge, «Dietary inhibitors of histone deacetylases in intestinal immunity and homeostasis,» Front Immunol, vol. 4, n. 226, pp. 1-7, 2013.
[33]R. Tao, E. de Zoeten, E. Ozkaynak e al, «Deacetylase Inhibition Promotes the Generation and Function of Regulatory T Cells,» Nat Med, vol. 13, n. 11, pp. 1299-1307, 2007.
[34]D. Bandarra, J. Biddlestone, S. Mudie e al, «HIF-1α restricts NF-κB-dependent gene expression to control innate immunity signals,» Dis Model Mech, vol. 8, n. 2, pp. 169-181, 2015.
[35]K. K. S. M. W. D. B. Krokowiczemail, «Sodium butyrate and short chain fatty acids in prevention of travellers' diarrhoea: A randomized prospective study,» 2014.
[36]S. K. K. K. W. e. a. Krokowicz, «Microencapsulated sodium butyrate administered to patients with diverticulosis decreases incidence of diverticulitisa prospective randomized study.,» 2014.
[37]P. Z. Kamath, «Short-Chain fatty acids stimulate ileal motility in humans.,» 1988.
[38]M. C. C. G. T. T. C. Di Sabotino, «Oral butyrate for mild to moderately active Crohn's disease.,» 2005.
[39]J. V. V. T. B. Hamer, «Review article: the role of butyrate on colonic function,» 2008.
[40]S. Petrosino e V. Di Marzo , «The pharmacology of palmitoyletathanolamide and first data on the therapeutic efficacy of some of its new formulations,» Br J Pharmacol, vol. 174, n. 11, pp. 1349-1365, 2017.
[41]J. LoVerme, G. La Rana, R. Russo e al, «The search for the palmitoylethanolamide receptor,» Life Sci, vol. 77, n. 14, pp. 1685-98, 2005.
[42]D. M. Petrosino, «The pharmacology of palmitoylethanolamide and first data on the therapeutic efficacy of some of its new formulations,» 2017.
[43]C. F. Jacobsson, «Characterization of palmitoylethanolamide transport in mouse Neuro-2a neuroblastoma and rat RBL-2H3 basophilic leukaemia cells: comparison with anandamide,» 2001.
[44]F. Borrelli, B. Romano, S. Petrosino e al, «palmitoylethanolamide, a naturally occurring lipid, is an orally effective intestinal anti-inflammatory agent,» Br J Pharmacol, vol. 172, n. 1, pp. 142-58, 2015.
[45]G. Esposito, E. Capoccia, F. Turco e al, «Palmitoyletanolamide improves colon inflammation through an enteric glia/toll like receptor 4-dependent PPAR-alpha activation,» Gut, vol. 63, n. 8, pp. 1300-1312, 2014.
[46]M. D. L. e. O. Couch, «The Use of Cannabinoids in Colitis: A Systematic Review and Meta-analysis,» 2018.
[47]C. Cremon, V. Stanghellini, M. Barbaro e al, «Randomised clinical trial: the analgesic properties of dietary supplementation with palmtoylethanolamide and polydatin in irritable bowel syndrome,» Aliment Pharmacol Ther, vol. 45, n. 7, pp. 909-922, 2017.
[48]B. Artukoglu, C. Beyer, A. Zuloff-Shani e al, «Efficacy of Palmitoylethanolamide for Pain: A Meta-Analysis.,» Pain Physician, vol. 20, n. 5, pp. 353-362, 2017.
[49]L. Van Kaer e D. Olivares-Villagómez, «Development, Homeostasis and Functions of Intestinal Intraepithelial Lymphocytes,» J Immunol, vol. 200, n. 7, pp. 2235-2244, 2018.
[50]G. Sarnelli, A. D'Alessandro, T. Iuvone e al, «Palmitoylethanolamide Modulates Inflamamtion-associated vascular endothelial growth factor (VEGF) signaling via the Akt/mTOR pathway in a Selective Peroxisome Proliferator-Activated Receptor Alpha (PPAR-alpha)-Dependent manner,» PLoS One, vol. 11, n. 5, 2016.
[51]D. Couch, H. Cook, C. Ortori e al, «Palmitoylethanolamide and Cannabidiol Prevent Inflammation induced Hyperpermeability of the Human Gut In Vitro and In Vivo-A Randomized, Placebo-controlled, Double-blind Controlled Trial,» Inflamm Bowel Dis, vol. 25, n. 6, pp. 1006-1018, 2019.
[52]Russo, R. e al: "Gut-brain Axis: Role of Lipids in the Regulation of Inflammation, Pain and CNS Diseases", CURRENT MEDICINAL CHEMISTRY, vol. 25, no. 32, 16 October 2018 (2018-10-16), pages 3930-3952.

## Claims

1. Combination for use in treating and preventing disorders and pathologies of the intestinal tract associated with or caused by inflammatory processes comprising the following active ingredients:
a) sodium butyrate;
b) palmitoylethanolamide (or PEA)
in which butyrate is administered at daily dosages between 400 and 3000 mg daily and PEA is administered at daily dosages between 200 and 1200 mg daily wherein the weight ratio between sodium butyrate and PEA administered daily is between 2:1 and 6:1.

2. Combination for use according to claim 1, wherein said disorders or pathologies of the intestinal tract are selected from: IBS (*Irritable Bowel syndrome*), inflammatory bowel disease IBD (*Inflammatory Bowel disease*), leaky bowel syndrome, celiac disease and food allergies.

3. Combination for use according to claim 1 or 2, wherein said weight ratio is comprised between 2:1 and 5:1, preferably 3:1.

4. Combination for use according to any one of claims 1 to 3, wherein said combination is administered in the form of two oral formulations, the first of which contains sodium butyrate as the active ingredient and the second formulation contains PEA as the active ingredient, or said combination is administered in the form of a single oral formulation which contains both active ingredients.

5. Combination for use according to claim 4, wherein said second oral formulation containing PEA also contains vitamin B2.

6. Combination for use according to claim 4 or 5, wherein said first formulation contains sodium butyrate in an amount of 900 mg and said second oral formulation contains PEA in quantities equal to 300 mg, and both are administered twice a day.

7. Combination for use according to any one of claims 4 to 6, wherein said oral formulations are food supplements or nutraceutical formulations.

8. Combination for use according to any one of claims 4 to 7, wherein said oral formulations are adjuvants.

9. Combination for use according to any one of claims 4 to 8, wherein said oral formulations are in the form of tablets, capsules, sachets, stickpacks in liquid or orosoluble or orodispersible powder form, bottles, ampoules.

## Patentansprüche

1. Kombination zur Verwendung bei der Behandlung und Vorbeugung von Erkrankungen und Pathologien des Darmtrakts, die mit entzündlichen Prozessen in Zusammenhang stehen oder durch diese verursacht werden, umfassend die folgenden Wirkstoffe:
a) Natriumbutyrat;
b) Palmitoylethanolamid (oder PEA)
wobei Butyrat in täglichen Dosierungen zwischen 400 und 3000 mg täglich und PEA in täglichen Dosierungen zwischen 200 und 1200 mg täglich verabreicht wird, wobei das Gewichtsverhältnis zwischen täglich verabreichtem Natriumbutyrat und PEA zwischen 2:1 und 6:1 liegt.

2. Kombination zur Verwendung nach Anspruch 1, wobei die Erkrankungen oder Pathologien des Darmtrakts ausgewählt sind aus: IBS (*Reizdarmsyndrom*), entzündliche Darmerkrankung IBD (*Inflammatory Bowel Disease*), Leaky Bowel Syndrom, Zöliakie und Nahrungsmittelallergien.

3. Kombination zur Verwendung nach Anspruch 1 oder 2, wobei das Gewichtsverhältnis zwischen 2:1 und 5:1, vorzugsweise 3:1, liegt.

4. Kombination zur Verwendung nach einem der Ansprüche 1 bis 3, wobei die Kombination in Form von zwei oralen Formulierungen verabreicht wird, von denen die erste Natriumbutyrat als Wirkstoff und die zweite PEA als Wirkstoff enthält, oder die Kombination in Form einer einzigen oralen Formulierung verabreicht wird, die beide Wirkstoffe enthält.

5. Kombination zur Verwendung nach Anspruch 4, wobei die zweite orale Formulierung, die PEA enthält, auch Vitamin B2 enthält.

6. Kombination zur Verwendung nach Anspruch 4 oder 5, wobei die erste Formulierung Natriumbutyrat in einer Menge von 900 mg enthält und die zweite orale Formulierung PEA in einer Menge von 300 mg enthält und beide zweimal täglich verabreicht werden.

7. Kombination zur Verwendung nach einem der Ansprüche 4 bis 6, wobei die oralen Formulierungen Nahrungsergänzungsmittel oder nutrazeutische Formulierungen sind.

8. Kombination zur Verwendung nach einem der Ansprüche 4 bis 7, wobei die oralen Formulierungen Adjuvantien sind.

9. Kombination zur Verwendung nach einem der Ansprüche 4 bis 8, wobei die oralen Formulierungen in Form von Tabletten, Kapseln, Beuteln, Stickpacks in flüssiger oder orosolöslicher oder orodispersibler Pulverform, Flaschen, Ampullen vorliegen.

## Revendications

1. Combinaison pour utilisation dans le traitement et la prévention de troubles et de pathologies du tractus intestinal associés ou causés par des processus inflammatoires comprenant les principes actifs suivants :
a) butyrate de sodium ;
b) palmitoyléthanolamide (ou PEA)
dans laquelle le butyrate est administré à des doses quotidiennes comprises entre 400 et 3 000 mg par jour et le PEA est administré à des doses quotidiennes comprises entre 200 et 1 200 mg par jour, dans laquelle le rapport pondéral entre le butyrate de sodium et le PEA administrés quotidiennement est compris entre 2:1 et 6:1.

2. Combinaison pour utilisation selon la revendication 1, dans laquelle lesdits troubles ou lesdites pathologies du tractus intestinal sont sélectionnés parmi : le SII (*syndrome de l'intestin irritable*), la maladie inflammatoire chronique de l'intestin MICI (*maladie inflammatoire chronique de l'intestin*), le syndrome de l'intestin perméable, la maladie coeliaque et les allergies alimentaires.

3. Combinaison pour utilisation selon la revendication 1 ou 2, dans laquelle ledit rapport pondéral est compris entre 2:1 et 5:1, de préférence 3:1.

4. Combinaison pour utilisation selon l'une quelconque des revendications 1 à 3, dans laquelle ladite combinaison est administrée sous la forme de deux formulations orales, dont la première contient du butyrate de sodium comme principe actif et la seconde formulation contient du PEA comme principe actif, ou ladite combinaison est administrée sous la forme d'une formulation orale unique qui contient les deux principes actifs.

5. Combinaison pour utilisation selon la revendication 4, dans laquelle ladite seconde formulation orale contenant du PEA contient également de la vitamine B2.

6. Combinaison pour utilisation selon la revendication 4 ou 5, dans laquelle ladite première formulation contient du butyrate de sodium en une quantité de 900 mg et ladite seconde formulation orale contient du PEA en des quantités égales à 300 mg, et les deux sont administrées deux fois par jour.

7. Combinaison pour utilisation selon l'une quelconque des revendications 4 à 6, dans laquelle lesdites formulations orales sont des compléments alimentaires ou des formulations nutraceutiques.

8. Combinaison pour utilisation selon l'une quelconque des revendications 4 à 7, dans laquelle lesdites formulations orales sont des adjuvants.

9. Combinaison pour utilisation selon l'une quelconque des revendications 4 à 8, dans laquelle lesdites formulations orales sont sous forme de comprimés, de capsules, de sachets, de sachets tubes sous forme liquide ou orosoluble ou pulvérulente orodispersible, de flacons, d'ampoules.
